# FASCICULE DE BREVET EUROPEEN

(11) **EP 3 534 813 B1**
(45) Date de publication et mention de la délivrance du brevet: **03.05.2023**
(21) Numéro de dépôt: 17797411.0
(22) Date de dépôt: 30.10.2017
(51) Int. Cl.: A61B 17/80, A61B 17/86

(54) **ENSEMBLE DE FIXATION POLYAXIALE D'OS**
MEHRACHSIGE KNOCHENFIXATIONSANORDNUNG
POLYAXIAL BONE FIXATION ASSEMBLY

(30) Priorité: 04.11.2016 FR 1660679
(43) Date de publication de la demande: 11.09.2019
(73) Titulaire: Neosteo, 44100 Nantes (FR)
(72) Inventeur: DECHELETTE, Maxime, 44390 Petit-Mars (FR); SORIN, Sylvain, 85530 La Bruffiere (FR)
(74) Mandataire: Ipsilon
(86) Numéro de dépôt international: PCT/FR2017/052982
(87) Numéro de publication internationale: WO 2018/083407

(56) Documents cités:
- US-A1- 2006 009 771
- US-A1- 2014 207 194
- US-A1- 2016 015 439
- US-B1- 8 652 183

## Description

### DOMAINE DE L'INVENTION

L'invention concerne un ensemble de fixation polyaxiale d'os.

Elle concerne plus particulièrement un ensemble de fixation poly-axiale d'os comprenant :
- un support de type plaque comprenant une face du dessus, une face du dessous et au moins un orifice traversant,
- au moins une vis de fixation de la plaque à une matière osseuse, cette vis étant composée d'une tête et d'un corps fileté, le corps de la vis, insérable depuis la face du dessus de la plaque dans l'orifice associé de la plaque, étant apte à traverser l'orifice pour venir se visser dans la matière osseuse et la tête étant apte à être retenue dans l'orifice associé de la plaque, par contact d'appui de surfaces sphériques complémentaires dites l'une, mâle, et portée par la tête, l'autre, femelle, et ménagée dans l'orifice associé de la plaque, cette surface sphérique femelle formant un siège à l'intérieur duquel la surface sphérique mâle est apte à prendre appui à l'état inséré de la vis dans l'orifice par la face du dessus de la plaque.

### ART ANTERIEUR

Parmi les moyens de fixer une ostéotomie ou une fracture osseuse, les systèmes plaque-vis à fixation verrouillée font l'objet d'un usage clinique répandu. Le principe de base est toujours le même : la vis s'ancre dans l'os via son filetage distal, tandis que le filetage de tête vient se bloquer dans un orifice traversant de la plaque. Ce blocage est appelé verrouillage.

Un exemple d'un tel système plaque-vis est décrit dans le brevet US 2014/0207194 ou le brevet US 2006/0009771.

Des exemples de vis sont également décrits dans US 8.652.183 et US 2016/0015439.

Il en résulte la constitution d'un ensemble monobloc plaque-vis qui améliore le maintien des fragments osseux, en particulier en fatigue. Les systèmes verrouillés sont ainsi moins sujets aux migrations des vis qui se désengagent de la plaque du fait des micromouvements. La consolidation osseuse est donc favorisée, puisque l'implant joue son rôle de tuteur osseux jusqu'à la consolidation partielle puis totale.

Il existe deux technologies de fixation verrouillée :
- la fixation monoaxiale : la vis ne peut être introduite que suivant un angle fixe, déterminé avec l'aide d'un canon de perçage qui coopère avec l'orifice de la plaque ;
- la fixation polyaxiale : la vis peut être insérée et verrouillée à la position angulaire désirée, comprise dans un cône de révolution ayant pour sommet le centre de rotation de l'orifice de la plaque.

Dans le cas de fractures ou d'ostéotomies complexes impliquant plusieurs fragments dont les localisations peuvent être variables, ou par nécessité d'encombrement (pour ne pas trop épaissir la plaque avec une fixation monoaxiale très angulée par rapport au reste de la plaque), l'usage d'une fixation polyaxiale est obligatoire.

La fixation polyaxiale peut être mise en oeuvre selon deux principes généraux :
- Le filet de tête de la vis vient coopérer avec le taraudage d'une pièce intermédiaire, montée solidaire de la plaque et disposant d'une mobilité permettant l'orientation de la vis une fois montée dans cette pièce intermédiaire. Il s'agit souvent d'une liaison rotule. Il en résulte un ensemble à 3 pièces comme l'illustrent par exemple, les brevets FR 2792185, ou US2011/0172666.

Le point faible de cette réalisation est, outre le nombre de pièces, l'épaississement de la plaque nécessaire pour loger la pièce intermédiaire. Il est aussi parfois possible d'expulser la pièce intermédiaire sous l'effet du couple de verrouillage.
- Le filet de tête de la vis vient coopérer avec un taraudage dans une plaque. Il n'y a pas de butée mécanique, les filets viennent se coincer entre eux par déformation, jusqu'au coincement complet du système comme l'illustre par exemple la demande internationale WO 2013/059090.

Le document US 2014/0207194 illustre également une telle solution.

Le point faible de cette réalisation est le phénomène de grippage (connu en orthopédie sous le nom de « cold welding », qui peut rendre impossible l'extraction de la vis, rallonger la durée de l'opération, provoquer des échauffements, nécroses osseuses et abandon de débris métalliques dans le corps du patient si la vis est extraite par perçage. Souvent, l'usage d'un tournevis dynamométrique permettant de stopper le verrouillage avant que le couple de grippage ne soit atteint doit être mis en oeuvre.

Cependant, ces instruments nécessitent entretien, calibration, et sont beaucoup plus onéreux à produire qu'un tournevis standard. De plus, en cas d'os scléreux, il est possible que le tournevis empêche prématurément le verrouillage de la vis.

En outre, les fixations réalisées suivant ce principe sont moins sécurisées en position monoaxiale (0 degré par rapport à l'axe de l'orifice), du fait de l'absence de butée. Il n'est ainsi pas rare de traverser la plaque avec la vis en tentant de la verrouiller.

De plus, dans les très grandes angulations, la tête de la vis a tendance à dépasser de la plaque.

Enfin, les filets étant détériorés de manière très importante, il n'est parfois pas possible de retirer puis de reverrouiller la vis dans le logement, à la différence d'une fixation avec correspondance entre filetage de tête et taraudage de plaque et disposant d'une butée.

Les problèmes rencontrés avec ces deux réalisations contribuent à la réputation des fixations polyaxiales reconnues comme moins solides que les fixations monoaxiales, en particulier pour le couple de verrouillage.

### BUTS ET RESUME

Un but de l'invention est de proposer un ensemble de fixation polyaxiale d'os dont la conception est en deux pièces sans nécessité d'outil dynamométrique pour le vissage.

A cet effet, l'invention a pour objet un ensemble de fixation poly-axiale d'os comprenant :
- un support de type plaque comprenant une face du dessus, une face du dessous et au moins un orifice traversant,
- au moins une vis de fixation de la plaque à une matière osseuse, cette vis étant composée d'une tête et d'un corps fileté, le corps de la vis, insérable depuis la face du dessus de la plaque dans l'orifice associé de la plaque, étant apte à traverser l'orifice pour venir se visser dans la matière osseuse et la tête étant apte à être retenue dans l'orifice associé de la plaque, par contact d'appui de surfaces sphériques complémentaires dites l'une, mâle, et portée par la tête, l'autre, femelle, et ménagée dans l'orifice associé de la plaque, cette surface sphérique femelle formant un siège à l'intérieur duquel la surface sphérique mâle est apte à prendre appui à l'état inséré de la vis dans l'orifice par la face du dessus de la plaque,
caractérisé en ce que l'alésage de l'orifice ou d'au moins l'un des orifices de la plaque apte à être associé à une vis de fixation comprend, depuis la face du dessous en direction de la face du dessus de la plaque, au moins une collerette interne, une portion cylindrique et la surface sphérique femelle formant siège, et en ce que la tête de la ou d'au moins l'une des vis de fixation associée comprend, entre sa surface sphérique mâle et sa zone de raccordement au corps de la vis, une portion filetée dont le filetage est à simple filet, ce filetage étant interrompu en au moins deux zones disposées à écartement l'une de l'autre sur le pourtour de la tête, chaque zone d'interruption du filetage ménageant au moins une arête de coupe apte à permettre, en parallèle de l'entrainement en rotation de la vis à l'intérieur dudit orifice jusqu'à une position en contact d'appui des surfaces sphériques mâle et femelle, un usinage, de préférence un taraudage, de la collerette interne de l'orifice associé de la plaque.

La présence d'une butée mécanique formée par le contact d'appui des surfaces sphériques mâle et femelle permet de contrôler de manière sûre le vissage résultant de la coopération de la portion filetée de la tête de vis et de la collerette de l'orifice. La vis est donc une vis compressive verrouillable dans la plaque. Cette vis permet, à l'état verrouillé dans la plaque, une mise en compression de la plaque sur la matière osseuse. En effet, la tête de vis est pourvue de deux zones distinctes, l'une à fonction de butée, l'autre à fonction de zone de prise avec la collerette. La tête de vis constitue donc à la fois un organe de verrouillage dans la plaque et un organe de compression de la plaque sur la matière osseuse indépendamment de sa position angulaire dans la plaque. La conception de l'ensemble permet en outre de monter et démonter plusieurs fois l'ensemble. Un tel ensemble peut également fonctionner avec des vis compressives non verrouillées.

Selon un mode de réalisation de l'invention, le filetage de la portion filetée de la tête de vis présente, depuis le sommet de la tête de vis en direction de sa zone de raccordement au corps de vis, un fond de filet définissant un corps de forme cylindrique puis un corps de forme conique.

Selon un mode de réalisation de l'invention, le filetage de la portion filetée de la tête de vis présente, depuis le sommet de la tête de vis en direction de sa zone de raccordement au corps de vis, une crête de filet définissant un corps de forme cylindrique ou cylindro-conique.

Selon un mode de réalisation de l'invention, le pas de la portion filetée de la tête de vis est inférieur ou égal au pas de la portion filetée du corps de vis.

Il en résulte une amélioration de l'engagement de la portion filetée de la vis dans la collerette interne de l'orifice associé de la plaque.

Selon un mode de réalisation de l'invention, la collerette interne de l'orifice de la plaque présente, à l'état usiné par la tête de la vis de fixation associée, une rainure hélicoïdale.

Selon un mode de réalisation de l'invention, chaque arête de coupe s'étend sur toute la hauteur de la portion filetée de la tête de vis.

Selon un mode de réalisation de l'invention, le diamètre du trou délimité par la collerette interne est inférieur au diamètre hors tout de la portion filetée de la tête de vis sur au moins une partie de la longueur de ladite portion filetée prise suivant l'axe longitudinal de la vis.

Selon un mode de réalisation de l'invention, la vis de fixation est, en position de fin de course à l'intérieur de l'orifice associé de la plaque, au niveau de la surface sphérique mâle de la tête de vis, en contact d'appui avec la surface sphérique femelle dudit orifice, et au niveau de la portion filetée de la tête de vis, en prise par vissage avec la collerette interne dudit orifice.

Selon un mode de réalisation de l'invention, la surface sphérique femelle formant siège de l'orifice ou d'au moins l'un des orifices de la plaque est une surface sphérique concave à concavité tournée vers la face du dessus de la plaque pour délimiter un logement de forme générale hémisphérique.

Selon un mode de réalisation de l'invention, le sommet de la tête de vis est muni d'une cavité axiale prismatique. Cette cavité sert à la réception d'un outil d'entraînement en rotation de la vis.

Selon un mode de réalisation de l'invention, la plaque et la ou au moins l'une des vis de fixation sont réalisées en un même matériau, de préférence en un même métal ou un même alliage métallique.

Selon un mode de réalisation de l'invention, la surface sphérique mâle portée par la tête est dimensionnée pour être logée, dans l'épaisseur de la plaque, à l'intérieur de l'orifice traversant de la plaque en position d'appui de fin de course des surfaces sphériques mâle de la tête et femelle de la plaque. Ce logement de la surface sphérique de la tête de vis dans l'épaisseur de la plaque est indépendant de la position angulaire de la vis à l'intérieur de l'orifice traversant de la plaque.

Selon un mode de réalisation de l'invention, l'épaisseur de la collerette est inférieure à 1 mm.

Selon un mode de réalisation de l'invention, l'épaisseur totale de la plaque est inférieure à 3 mm.

### BREVE DESCRIPTION DES DESSINS

L'invention sera bien comprise à la lecture de la description suivante d'exemples de réalisation, en référence aux dessins annexés dans lesquels :

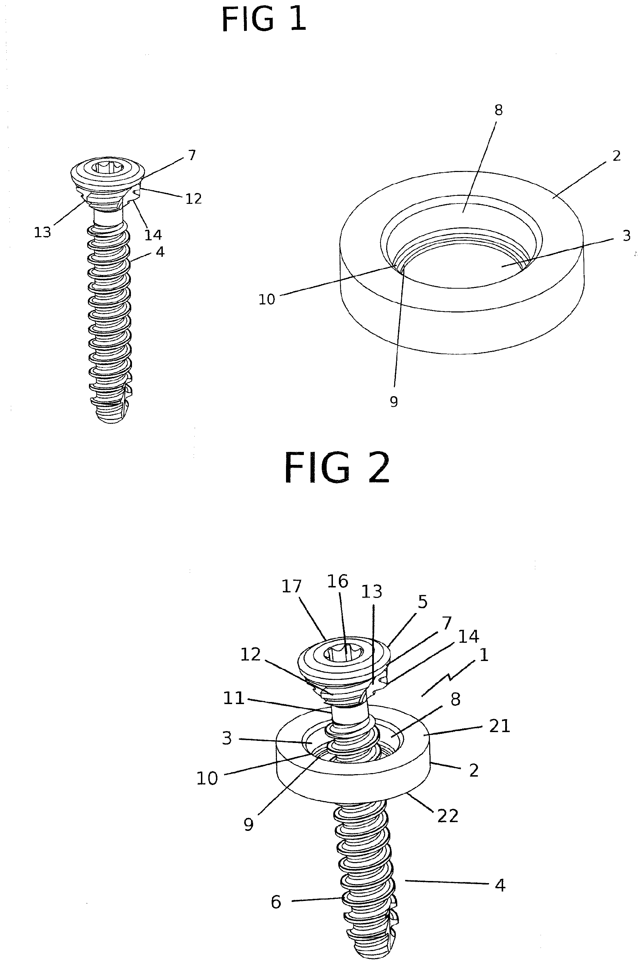

La figure 1 représente une vue en perspective d'une vis et d'une plaque à un orifice traversant,
La figure 2 représente une vue en perspective d'une vis à l'état inséré partiellement dans l'orifice d'une plaque,

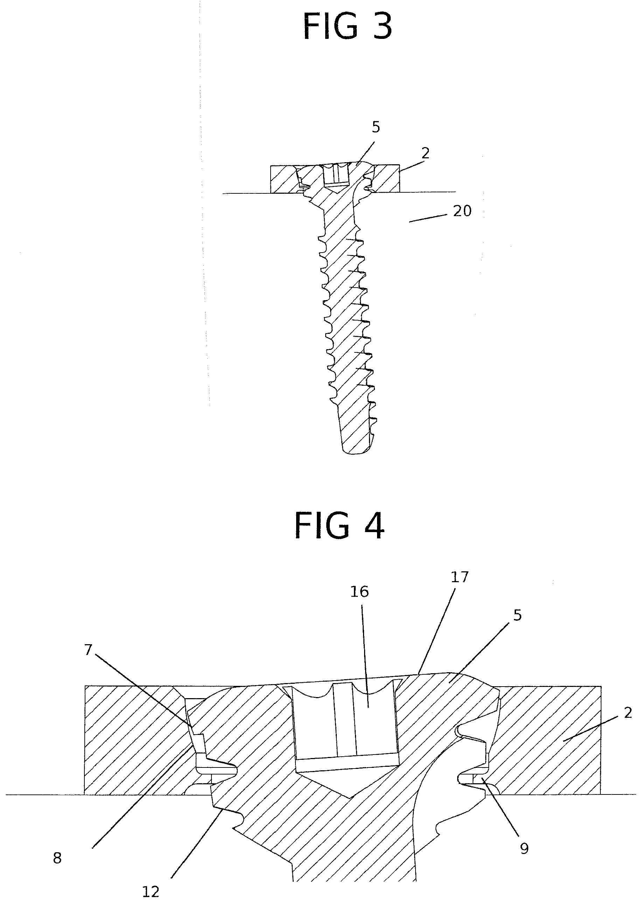

La figure 3 représente une vue en coupe d'une vis à l'état inséré partiellement dans l'orifice d'une plaque, lors d'une opération de vissage,
La figure 4 représente une vue de détail agrandie de la partie fixation de la tête de la figure 3,

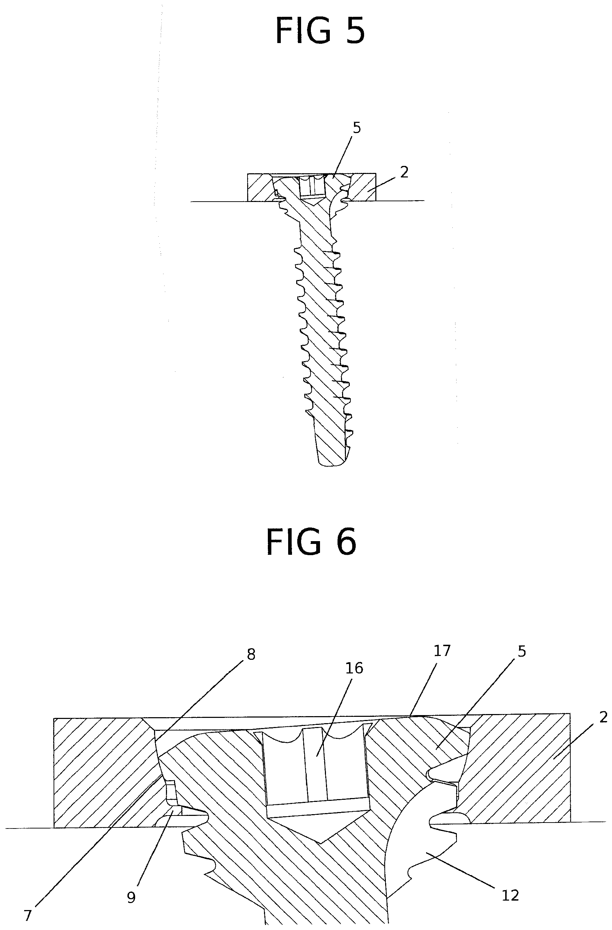

La figure 5 représente une vue en coupe d'une vis à l'état inséré totalement dans l'orifice d'une plaque, lors d'une opération de vissage, les surfaces sphériques mâle et femelle étant en contact d'appui et la collerette interne ayant été taraudée,
La figure 6 représente une vue de détail agrandie de la partie fixation de la tête de la figure 5,

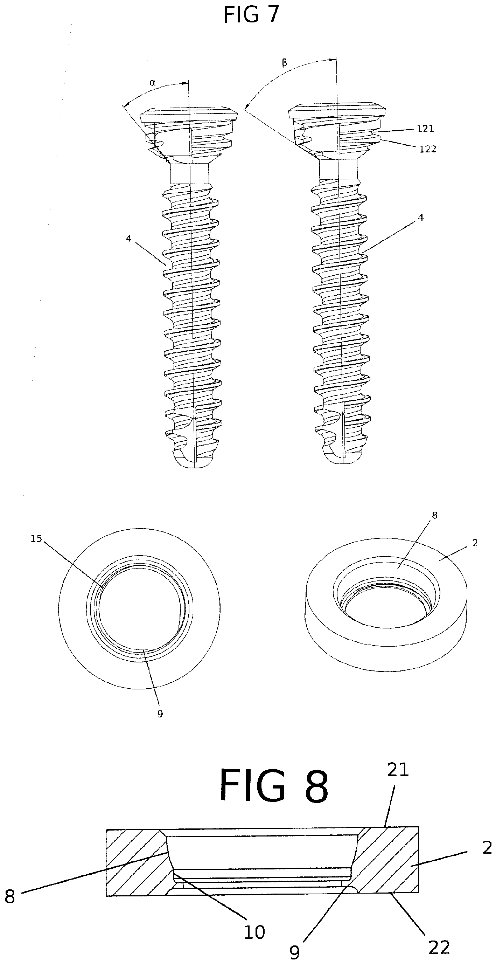

La figure 7 représente une vue schématique de deux vis et de deux plaques à l'état taraudé de la collerette,
La figure 8 représente une vue en coupe d'une plaque avant taraudage de la collerette interne,
La figure 9 représente une vue en coupe d'une vis à l'état inséré partiellement dans l'orifice d'une plaque, lors d'une opération de vissage, ladite vis étant une vis compressive.

### DESCRIPTION DETAILLEE

Comme mentionné ci-dessus, l'invention a pour objet un ensemble 1 de fixation poly-axiale d'os comprenant une plaque 2 positionnable en appui sur l'os et au moins une vis 4 de fixation de la plaque 2 à l'os. La plaque 2 peut affecter un grand nombre de formes, plane, en T, en L ou autre. Dans l'exemple représenté, cette plaque 2 est une plaque plane réalisée en un alliage métallique qui est le même que celui utilisé pour la vis 4 de fixation. En variante, la plaque 2 aurait pu être réalisée en un alliage métallique différent de celui utilisé pour la vis 4 de fixation.

La plaque 2 comprend une face 21 du dessus, une face 22 du dessous opposée à la face du dessus et positionnable en applique sur l'os et au moins un orifice 3 traversant. Dans l'exemple représenté, la plaque 2 ne comporte qu'un seul orifice 3 traversant pour des raisons de simplification des dessins. Toutefois, elle peut comporter plusieurs orifices sans sortir du cadre de l'invention.

La vis 4 de fixation de la plaque 2 à une matière 20 osseuse est composée d'une tête 5 et d'un corps 6 fileté. Ce corps 6 est raccordé à la tête 5 par une zone 11 de raccordement qui est ici non filetée et représentée cylindrique. Le corps 6 de la vis 4 est insérable depuis la face 21 du dessus de la plaque 2 dans l'orifice 3 traversant de la plaque 2 pour venir se visser dans la matière 20 osseuse et la tête 5 est apte à être retenue dans l'orifice 3 de la plaque 2 par contact d'appui de surfaces 7, 8 sphériques complémentaires dites l'une 7, mâle, et portée par la tête 5, l'autre 8, femelle, et ménagée dans l'orifice 3 de la plaque 2. Ces surfaces sphériques mâle 7 et femelle 8 complémentaires sont, dans leurs zones aptes à venir en contact d'appui des surfaces lisses. Ces surfaces sphériques mâle et femelle complémentaires sont donc, dans leurs zones aptes à venir en contact d'appui à l'état inséré de la vis dans l'orifice associé de la plaque, dépourvues de filetage. La surface 8 sphérique femelle forme un siège à l'intérieur duquel la surface 7 sphérique mâle est apte à prendre appui à l'état inséré de la vis 4 dans l'orifice 3 par la face 21 du dessus de la plaque 2. Cette surface 8 sphérique femelle est une surface sphérique concave à concavité tournée vers la face 21 du dessus de la plaque 2 pour délimiter un logement de forme générale hémisphérique. Le rayon de cette surface 8 sphérique femelle est sensiblement égal au rayon de la surface sphérique mâle au jeu près nécessaire à l'insertion de la surface sphérique femelle dans la surface sphérique mâle. La présence de surfaces sphériques permet une orientation angulaire quelconque de la vis à l'intérieur de l'orifice 3 de la plaque tout en maintenant un contact d'appui entre lesdites surfaces.

L'alésage de l'orifice 3 traversant de la plaque 2 comprend encore depuis la face 21 du dessus en direction de la face 22 du dessous dans le prolongement de la surface 8 sphérique femelle, une portion cylindrique ou lamage, puis une collerette 9 interne ou lèvre circulaire radiale interne dont le rôle sera décrit ci-après. L'épaisseur de cette collerette 9 est généralement inférieure à 1 mm et de préférence voisine de 0,2 mm. L'épaisseur totale de la plaque 2 est de préférence inférieure à 3 mm.

Le sommet 17 de la tête 5 de vis est muni d'une cavité 16 axiale prismatique servant à la réception d'un outil d'entrainement en rotation de la vis. La tête 5 de la vis 4 de fixation comprend, entre sa surface 7 sphérique mâle et sa zone 11 de raccordement au corps 6 de la vis 4, une portion 12 filetée. Le filetage de cette portion 12 filetée de la tête 5 de vis présente, depuis le sommet 17 de la tête 5 de vis en direction de sa zone 11 de raccordement au corps 6 de vis, un fond 121 de filet définissant un corps de forme cylindrique puis un corps de forme conique. L'angle α formé par le fond de filet de la portion conique avec l'axe longitudinal de la vis est de préférence compris entre 5° et 60°. Le filetage de la portion 12 filetée de la tête 5 de vis présente, depuis le sommet 17 de la tête 5 de vis en direction de sa zone 11 de raccordement au corps 6 de vis, une crête 122 de filet définissant un corps de forme cylindrique ou cylindro-conique. L'angle β formé par la crête de filet de la portion conique avec l'axe longitudinal de la vis est de préférence compris entre 5° et 80°. Le pas de la portion 12 filetée de la tête 5 de vis est inférieur ou égal au pas de la portion filetée du corps 6 de vis. Il en résulte un placage de la plaque contre l'os et un meilleur engagement de la portion filetée de la vis dans la collerette, cet engagement étant décrit ci-après. Le filetage de cette portion 12 filetée de la tête 5 de vis est à simple filet. Ce filetage est interrompu en au moins deux zones 13 disposées à écartement l'une de l'autre sur le pourtour de la tête 5. Dans l'exemple représenté, ces zones 13 d'interruption sont au nombre de 3 et sont écartées l'une de l'autre de 120°. En présence de deux zones d'interruption, ces dernières auraient été ménagées de préférence de manière diamétralement opposée. Indépendamment du nombre de zones d'interruption, chaque zone 13 d'interruption du filetage ménage au moins une arête 14 de coupe apte à permettre, en parallèle de l'entrainement en rotation de la vis à l'intérieur dudit orifice jusqu'à une position en contact d'appui des surfaces sphériques mâle 7 et femelle 8, un taraudage, de la collerette 9 interne de l'orifice 3 de la plaque 2. Ainsi, la collerette 9 de l'orifice 3 de la plaque 2 présente, à l'état usiné par la tête de la vis de fixation associée qui fait office de taraud, une rainure 15 hélicoïdale. La vis 4 de fixation est donc, en position de fin de course à l'intérieur de l'orifice 3 de la plaque 2, au niveau de la surface 7 sphérique mâle de la tête de vis, en contact d'appui avec la surface 8 sphérique femelle dudit orifice 3, et au niveau de la portion 12 filetée de la tête 5 de vis, en prise par vissage avec la collerette 9 interne dudit orifice 3. Pour permettre un tel usinage de la collerette par la portion filetée de la vis, le diamètre du trou délimité par la collerette 9 interne est inférieur au diamètre hors tout de la portion 12 filetée de la tête 5 de vis sur au moins une partie de la longueur de ladite portion 12 filetée prise suivant l'axe longitudinal de la vis 4.

Dans l'exemple représenté, chaque arête 14 de coupe s'étend sur toute la hauteur de la portion 12 filetée de la tête 5 de vis. De même, dans l'exemple représenté, la tête de vis comprend entre son sommet et sa portion 12 filetée un renflement ou protubérance ou excroissance radiale externe formant une collerette périphérique externe, le bord circonférentiel externe de la collerette formant la surface 7 sphérique mâle constituée donc par la surface externe convexe de la portion de sphère prise entre deux plans parallèles orthogonaux à l'axe longitudinal de la vis.

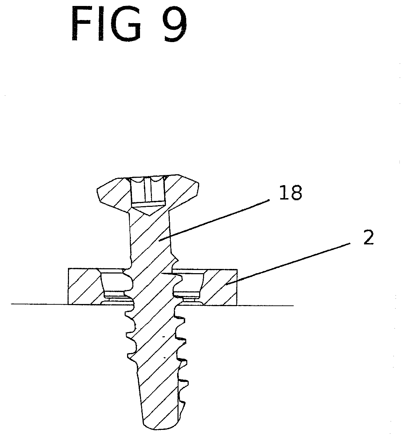

La figure 9 illustre un autre type de vis représentée en 18 et utilisable avec la plaque en particulier quand la plaque présente plusieurs orifices 3 traversants. Cette vis se distingue de la vis représentée aux autres figures par l'absence de portion filetée au niveau de la tête. Cette vis est donc une simple vis compressive et non une vis verrouillée comme dans les autres exemples. Cette figure illustre le fait que le dispositif peut comprendre en sus d'au moins une vis de fixation dite verrouillée une vis de fixation dite compressive se distinguant de la vis de fixation verrouillée par l'absence de portion filetée au niveau de la tête de vis.

La fixation d'un tel dispositif s'opère donc comme suit : la plaque est positionnée en applique sur l'os. Le chirurgien procède à travers la plaque à un perçage dans l'os à l'aide d'un canon de perçage. L'axe du perçage est choisi pour obtenir une orientation de la vis telle qu'elle résiste à des forces d'arrachement importantes. La vis est alors introduite à travers la plaque dans l'axe du perçage et vissée. Au cours du vissage, la portion 12 filetée de la vis vient tarauder la collerette 9 interne générant une rainure hélicoïdale dans ladite collerette 9. Le vissage est poursuivi jusqu'à une mise en butée obtenue par coopération des surfaces sphériques mâle et femelle. Lors de la poursuite du vissage après mise en butée, la portion filetée de la tête de vis vient effectuer un coincement avec la collerette interne, ce qui décuple l'effet et la sensation de verrouillage. La portion 10 cylindrique de l'alésage de l'orifice est configurée, c'est-à-dire conformée et dimensionnée, pour éviter tout contact prématuré entre la partie tranchante de la portion filetée de la tête de vis et la collerette 9 interne avant la mise en butée par contact d'appui des surfaces sphériques de manière à assurer un taraudage de la collerette en fin de course de la tête de vis à l'intérieur de l'orifice de la plaque juste avant mise en butée.

La conception de l'ensemble tel que décrit ci-dessus permet de procéder à plusieurs vissages/dévissages de la vis offrant la possibilité au chirurgien de démonter la vis si nécessaire et de la réintroduire, y compris suivant un axe différent de celui de la première insertion.

## Revendications

1. Ensemble (1) de fixation poly-axiale d'os comprenant :
- un support de type plaque (2) comprenant une face (21) du dessus, une face (22) du dessous et au moins un orifice (3) traversant,
- au moins une vis (4) de fixation de la plaque (2) à une matière (20) osseuse, cette vis (4) étant composée d'une tête (5) et d'un corps (6) fileté, le corps (6) de la vis (4), insérable depuis la face (21) du dessus de la plaque (2) dans l'orifice (3) associé de la plaque (2), étant apte à traverser l'orifice (3) pour venir se visser dans la matière (20) osseuse et la tête (5) étant apte à être retenue dans l'orifice (3) associé de la plaque (2), par contact d'appui de surfaces (7, 8) sphériques complémentaires dites l'une (7), mâle, et portée par la tête (5), l'autre (8), femelle, et ménagée dans l'orifice (3) associé de la plaque (2) , cette surface (8) sphérique femelle formant un siège à l'intérieur duquel la surface (7) sphérique mâle est apte à prendre appui à l'état inséré de la vis (4) dans l'orifice (3) par la face (21) du dessus de la plaque (2),
**caractérisé en ce que** l'alésage de l'orifice (3) ou d'au moins l'un des orifices (3) de la plaque (2), apte à être associé à une vis (4) de fixation comprend, depuis la face (22) du dessous en direction de la face (21) du dessus de la plaque(2), au moins une collerette (9) interne, une portion (10) cylindrique et la surface (8) sphérique femelle formant siège, et **en ce que** la tête (5) de la, ou d'au moins l'une des vis (4) de fixation associée comprend, entre sa surface (7) sphérique mâle et sa zone (11) de raccordement au corps (6) de la vis (4), une portion (12) filetée dont le filetage est à simple filet, ce filetage étant interrompu en au moins deux zones (13) disposées à écartement l'une de l'autre sur le pourtour de la tête (5), chaque zone (13) d'interruption du filetage ménageant au moins une arête (14) de coupe apte à permettre, en parallèle de l'entrainement en rotation de la vis (4) à l'intérieur dudit orifice (3) jusqu'à une position en contact d'appui des surfaces sphériques mâle (7) et femelle (8), un usinage, de préférence un taraudage, de la collerette (9) interne de l'orifice (3) associé de la plaque (2) .

2. Ensemble (1) de fixation poly-axiale d'os selon la revendication 1,
**caractérisé en ce que** le filetage de la portion (12) filetée de la tête (5) de vis présente, depuis le sommet (17) de la tête (5) de vis en direction de sa zone (11) de raccordement au corps (6) de vis, un fond (121) de filet définissant un corps de forme cylindrique puis un corps de forme conique.

3. Ensemble (1) de fixation poly-axiale d'os selon l'une des revendications précédentes,
**caractérisé en ce que** le filetage de la portion (12) filetée de la tête (5) de vis présente, depuis le sommet (17) de la tête (5) de vis en direction de sa zone (11) de raccordement au corps (6) de vis, une crête (122) de filet définissant un corps de forme cylindrique ou cylindro-conique.

4. Ensemble (1) de fixation poly-axiale d'os selon l'une des revendications précédentes,
**caractérisé en ce que** le pas de la portion (12) filetée de la tête (5) de vis est inférieur ou égal au pas de la portion filetée du corps (6) de vis.

5. Ensemble (1) de fixation poly-axiale d'os selon l'une des revendications précédentes, **caractérisé en ce que** la collerette (9) interne de l'orifice (3) de la plaque (2) présente, à l'état usiné par la tête de la vis de fixation associée, une rainure (15) hélicoïdale.

6. Ensemble (1) de fixation poly-axiale d'os selon l'une des revendications précédentes,
**caractérisé en ce que** chaque arête (14) de coupe s'étend sur toute la hauteur de la portion (12) filetée de la tête (5) de vis.

7. Ensemble (1) de fixation poly-axiale d'os selon l'une des revendications précédentes,
**caractérisé en ce que** le diamètre du trou délimité par la collerette (9) interne est inférieur au diamètre hors tout de la portion (12) filetée de la tête (5) de vis sur au moins une partie de la longueur de ladite portion (12) filetée prise suivant l'axe longitudinal de la vis (4).

8. Ensemble (1) de fixation poly-axiale d'os selon l'une des revendications précédentes,
**caractérisé en ce que** la vis (4) de fixation est, en position de fin de course à l'intérieur de l'orifice (3) associé de la plaque (2), au niveau de la surface (7) sphérique mâle de la tête de vis, en contact d'appui avec la surface (8) sphérique femelle dudit orifice (3), et au niveau de la portion (12) filetée de la tête (5) de vis, en prise par vissage avec la collerette (9) interne dudit orifice (3).

9. Ensemble (1) de fixation poly-axiale d'os selon l'une des revendications précédentes,
**caractérisé en ce que** la surface (8) sphérique femelle formant siège de l'orifice (3) ou d'au moins l'un des orifices (3) de la plaque (2) est une surface sphérique concave à concavité tournée vers la face (21) du dessus de la plaque (2) pour délimiter un logement de forme générale hémisphérique.

10. Ensemble (1) de fixation poly-axiale d'os selon l'une des revendications précédentes,
**caractérisé en ce que** le sommet (17) de la tête (5) de vis est muni d'une cavité (16) axiale prismatique.

11. Ensemble (1) de fixation poly-axiale d'os selon l'une des revendications précédentes,
**caractérisé en ce que** la plaque (2) et la ou au moins l'une des vis (4) de fixation sont réalisées en un même matériau, de préférence en un même métal ou un même alliage métallique.

12. Ensemble (1) de fixation poly-axiale d'os selon l'une des revendications précédentes,
**caractérisé en ce que** la surface sphérique mâle (7) portée par la tête (5) de vis est dimensionnée pour être logée, dans l'épaisseur de la plaque (2), à l'intérieur de l'orifice (3) traversant de la plaque (2) en position d'appui de fin de course des surfaces sphériques mâle (7) de la tête de vis et femelle (8) de la plaque (2).

13. Ensemble (1) de fixation poly-axiale d'os selon l'une des revendications précédentes,
**caractérisé en ce que** l'épaisseur de la collerette (9) est inférieure à 1 mm.

14. Ensemble (1) de fixation poly-axiale d'os selon l'une des revendications précédentes,
**caractérisé en ce que** l'épaisseur totale de la plaque (2) est inférieure à 3 mm.

## Patentansprüche

1. Mehrachsige Knochenfixationsanordnung (1) umfassend:
- einen Träger vom Typ Platte (2), umfassend eine obere Seite (21), eine untere Seite (22) und mindestens eine Durchgangsöffnung (3),
- mindestens eine Schraube (4) zur Befestigung der Platte (2) an ein Knochenmaterial (20), wobei diese Schraube (4) aus einem Kopf (5) und einem gewindegeschnittenen Körper (6) zusammengesetzt ist, wobei der Körper (6) der Schraube (4), der von der unteren Seite (21) der Platte (2) in die Öffnung (3) eingeführt werden kann, die mit der Platte (2) assoziiert ist, ausgelegt ist, um die Öffnung (3) zu queren, um in das Knochenmaterial (20) eingeschraubt zu werden, und wobei der Kopf (5) ausgelegt ist, um in der Öffnung (3), die mit der Platte (2) assoziiert ist, durch Auflagekontakt von komplementären sphärischen Flächen (7, 8) zurückgehalten zu werden, von denen eine (7) als Außenfläche und vom Kopf (5) getragen, die andere (8) als Innenfläche und bereitgestellt in der Öffnung (3) bezeichnet wird, die mit der Platte (2) assoziiert ist, wobei diese sphärische Innenfläche (8) einen inneren Sitz bildet, dessen sphärische Außenfläche (7) ausgelegt ist, um im eingeführten Zustand der Schraube (4) in die Öffnung (3) durch die untere Seite (21) der Platte (2) in Auflage zu kommen,
**dadurch gekennzeichnet, dass** die Bohrung der Öffnung (3) oder mindestens einer der Öffnungen (3) der Platte (2), die ausgelegt ist, um mit einer Befestigungsschraube (4) assoziiert zu sein, von der Unterseite (22) in Richtung der Oberseite (21) der Platte (2) mindestens einen internen Kragen (9) umfasst, wobei ein zylindrischer Abschnitt (10) und die sphärische Innenläche (8) einen Sitz bilden, und dadurch, dass der Kopf (5) der oder mindestens einer der assoziierten Befestigungsschrauben (4) zwischen seiner sphärischen Außenfläche (7) und seinem Bereich (11) der Verbindung an den Körper (6) der Schraube (4) einen gewindegeschnittenen Abschnitt (12) umfasst, dessen Gewinde einen einfachen Gewindegang aufweist, wobei dieses Gewinde in mindestens zwei Bereichen (13) unterbrochen ist, die mit Beabstandung voneinander auf dem Umfang des Kopfs (5) angeordnet sind, wobei jeder Unterbrechungsbereich (13) des Gewindes mindestens eine Schnittkante (14) bereitstellt, die ausgelegt ist, um parallel mit der Versetzung der Schraube in Rotation (4) im Inneren der Öffnung (3) bis in eine Position in Auflagekontakt der sphärischen Außen- (7) und Innenflächen (8) eine Bearbeitung, vorzugsweise eine Bohrung, des internen Kragens (9) der Öffnung (3) zu ermöglichen, die mit der Platte (2) assoziiert ist.

2. Mehrachsige Knochenfixationsanordnung (1) nach Anspruch 1, **dadurch gekennzeichnet, dass** das Gewinde des gewindegeschnittenen Abschnitts (12) des Schraubenkopfs (5) von der Spitze (17) des Schraubenkopfs (5) in Richtung seines Bereichs (11) der Verbindung mit dem Schraubenkörper (6) einen Gewindeboden (121), der einen Körper in zylindrischer Form, dann einen Körper in konischer Form definiert.

3. Mehrachsige Knochenfixationsanordnung (1) nach einem der vorhergehenden Ansprüche, **dadurch gekennzeichnet, dass**
das Gewinde des gewindegeschnittenen Abschnitts (12) des Schraubenkopfs (5) von der Spitze (17) des Schraubenkopfs (5) in Richtung seines Bereichs (11) der Verbindung mit dem Schraubenkörper (6) eine Gewindesteigung (122) aufweist, die einen Körper in zylindrischer oder zylindrisch-konischer Form aufweist.

4. Mehrachsige Knochenfixationsanordnung (1) nach einem der vorhergehenden Ansprüche, **dadurch gekennzeichnet, dass**
der Gang des gewindegeschnittenen Abschnitts (12) des Schraubenkopfs (5) kleiner als oder gleich wie der Gang des gewindegeschnittenen Abschnitts des Körpers (6) der Schraube ist.

5. Mehrachsige Knochenfixationsanordnung (1) nach einem der vorhergehenden Ansprüche, **dadurch gekennzeichnet, dass** der interne Kragen (9) der Öffnung (3) der Platte (2) im bearbeiteten Zustand durch den Kopf der assoziierten Befestigungsschraube eine spiralförmigen Nut (15) aufweist.

6. Mehrachsige Knochenfixationsanordnung (1) nach einem der vorhergehenden Ansprüche, **dadurch gekennzeichnet, dass**
sich jede Schneidkante (14) über die gesamte Höhe des gewindegeschnittenen Abschnitts (12) des Schraubenkopfs (5) erstreckt.

7. Mehrachsige Knochenfixationsanordnung (1) nach einem der vorhergehenden Ansprüche, **dadurch gekennzeichnet, dass**
der Durchmesser des Lochs, begrenzt durch den internen Kragen (9), kleiner als der Gesamtdurchmesser des gewindegeschnittenen Abschnitts (12) des Schraubenkopfs (5) auf mindestens einem Teil der Länge des gewindegeschnittenen Abschnitts (12) ist, gesehen gemäß der Längsachse der Schraube (4).

8. Mehrachsige Knochenfixationsanordnung (1) nach einem der vorhergehenden Ansprüche, **dadurch gekennzeichnet, dass**
die Befestigungsschraube (4) in der Endposition im Inneren der Öffnung (3), die mit der Platte (2) assoziiert ist, auf der Ebene der sphärischen Außenfläche (7) des Schraubenkopfs, in Auflagekontakt mit der sphärische Innenfläche (8) der Öffnung (3), und auf der Ebene des gewindegeschnittenen Abschnitts (12) des Schraubenkopfs (5), in Eingriff durch Verschraubung mit dem internen Kragen (9) der Öffnung (3) ist.

9. Mehrachsige Knochenfixationsanordnung (1) nach einem der vorhergehenden Ansprüche, **dadurch gekennzeichnet, dass**
die sphärische Innenfläche (8), die einen Sitz der Öffnung (3) oder mindestens einer der Öffnungen (3) der Platte (2) bildet, eine konkave sphärische Fläche ist, mit einer Konkavität, die gegen die Unterseite (21) der Platte (2) gedreht ist, um eine Aufnahme mit im Allgemeinen halbsphärischer Form zu begrenzen.

10. Mehrachsige Knochenfixationsanordnung (1) nach einem der vorhergehenden Ansprüche, **dadurch gekennzeichnet, dass**
die Spitze (17) des Schraubenkopfs (5) mit einem prismatischen axialen Hohlraum (16) ausgestattet ist.

11. Mehrachsige Knochenfixationsanordnung (1) nach einem der vorhergehenden Ansprüche, **dadurch gekennzeichnet, dass**
die Platte (2) und die oder mindestens eine der Befestigungsschrauben (4) aus einem gleichen Material hergestellt sind, vorzugsweise einem gleichen Metall oder einer gleichen metallischen Legierung.

12. Mehrachsige Knochenfixationsanordnung (1) nach einem der vorhergehenden Ansprüche, **dadurch gekennzeichnet, dass**
die sphärische Außenfläche (7), getragen vom Schraubenkopf (5), abgemessen ist, um in der Dicke der Platte (2), im Inneren der Durchgangsöffnung (3) der Platte (2) in der Endposition der sphärischen Außenfläche (7) des Schraubenkopfs und Innenfläche (8) der Platte (2) aufgenommen zu sein.

13. Mehrachsige Knochenfixationsanordnung (1) nach einem der vorhergehenden Ansprüche, **dadurch gekennzeichnet, dass**
die Dicke des Kragens (9) kleiner als 1 mm ist.

14. Mehrachsige Knochenfixationsanordnung (1) nach einem der vorhergehenden Ansprüche, **dadurch gekennzeichnet, dass**
die Gesamtdicke des Kragens (2) kleiner als 3 mm ist.

## Claims

1. A polyaxial bone fastener assembly (1) comprising:
- a plate-type support (2) having a top face (21), a bottom face (22) and at least one through orifice (3),
- at least one fastener screw (4) for fastening the plate (2) to bone material (20), this screw (4) comprising a head (5) and a threaded body (6), the body (6) of the screw (4) being insertable from the top face (21) of the plate (2) into the associated orifice (3) in the plate (2) and being suitable for passing through the orifice (3) in order to be screwed into the bone material (20), and the head (5) being suitable for being held in the associated orifice (3) in the plate (2) by bearing contact between complementary spherical surfaces (7, 8), one of which (7) is said to be "male" and carried by the head (5) and the other of which (8) is said to be "female" and is provided in the associated orifice (3) in the plate (2), this female spherical surface (8) forming a seat within which the male spherical surface (7) is suitable for bearing when the screw (4) is inserted in the orifice (3) through the top face (21) of the plate (2),
**characterized in that** the bore of the orifice (3) or of at least one of the orifices (3) in the plate (2) and that is suitable for being associated with a fastener screw (4) comprises, going from the bottom face (22) toward the top face (21) of the plate (2), at least one inner collar (9), a cylindrical portion (10), and the seat-forming female spherical surface (8), and **in that** the head (5) of the associated fastener screw (4), or of at least one of the associated fastener screws, comprises, between its male spherical surface (7) and a zone (11) for connection to the body (6) of the screw (4), a threaded portion (12) having a single-start thread, this thread being interrupted in at least two zones (13) that are spaced apart from each other around the circumference of the head (5), each interruption zone (13) in the thread providing at least one cutting edge (14) suitable for machining, preferably tapping, the inner collar (9) in the associated orifice (3) of the plate (2) while the screw (4) is being driven to turn inside said orifice (3) until reaching a position of bearing contact between the male (7) and female (8) spherical surfaces.

2. The polyaxial bone fastener assembly (1) according to claim 1,
**characterized in that** the thread of the threaded portion (12) of the screw head (5) presents, going from the top (17) of the screw head (5) toward its zone (11) connected to the screw body (6), a thread root (121) defining a body of cylindrical shape followed by a body of conical shape.

3. The polyaxial bone fastener assembly (1) according to one of the preceding claims,
**characterized in that** the thread of the threaded portion (12) of the screw head (5) presents, going from the top (17) of the screw head (5) toward its zone (11) connected to the screw body (6), a thread crest (122) defining a body of cylindrical shape or of cylindrical-conical shape.

4. The polyaxial bone fastener assembly (1) according to one of the preceding claims,
**characterized in that** the pitch of the threaded portion (12) of the screw head (5) is less than or equal to the pitch of the threaded portion of the screw body (6).

5. The polyaxial bone fastener assembly (1) according to one of the preceding claims,
**characterized in that** the inner collar (9) of the orifice (3) in the plate (2) presents, once machined by the head of the associated fastener screw, a helical groove (15).

6. The polyaxial bone fastener assembly (1) according to one of the preceding claims,
**characterized in that** each cutting edge (14) extends over the full height of the threaded portion (12) of the screw head (5).

7. The polyaxial bone fastener assembly (1) according to one of the preceding claims,
**characterized in that** the diameter of the hole defined by the inner collar (9) is less than the overall diameter of the threaded portion (12) of the screw head (5) over at least a fraction of the length of said threaded portion (12) measured along the longitudinal axis of the screw (4).

8. The polyaxial bone fastener assembly (1) according to one of the preceding claims,
**characterized in that** the fastener screw (4), in its end-of-stroke position inside the associated orifice (3) of the plate (2), is in bearing contact via the male spherical surface (7) of the screw head with the female spherical surface (8) of said orifice (3), and is in screw engagement via the threaded portion (12) of the screw head (5) with the inner collar (9) of said orifice (3).

9. The polyaxial bone fastener assembly (1) according to one of the preceding claims,
**characterized in that** the seat-forming female spherical surface (8) of the orifice (3), or of at least one of the orifices (3), in the plate (2) is a concave spherical surface with its concave side facing toward the top face (21) of the plate (2) in order to define a housing of generally hemispherical shape.

10. The polyaxial bone fastener assembly (1) according to one of the preceding claims,
**characterized in that** the top (17) of the screw head (5) is provided with a prismatic axial socket (16).

11. The polyaxial bone fastener assembly (1) according to one of the preceding claims,
**characterized in that** the plate (2) and the or at least one of the fastener screw(s) (4) are made of the same material, preferably the same metal or the same metal alloy.

12. The polyaxial bone fastener assembly (1) according to one of the preceding claims,
**characterized in that** the male spherical surface (7) carried by the screw head (5) is dimensioned to be received in the thickness of the plate (2) inside the through orifice (3) in the plate (2) in an end-of-stroke bearing position between the male spherical surface (7) of the screw head and the female spherical surface (8) of the plate (2).

13. The polyaxial bone fastener assembly (1) according to one of the preceding claims, **characterized in that** the thickness of the collar (9) is less than 1 mm.

14. The polyaxial bone fastener assembly (1) according to one of the preceding claims, **characterized in that** the total thickness of the plate (2) is less than 3 mm.
